# FASCICULE DE BREVET EUROPEEN

(11) **EP 1 633 392 B1**
(45) Date de publication et mention de la délivrance du brevet: **30.03.2016**
(21) Numéro de dépôt: 04742551.7
(22) Date de dépôt: 20.04.2004
(51) Int. Cl.: A61K 38/28, A61K 38/00, A61K 9/48, A61K 9/50, A61K 9/51, A61K 9/16, A61K 9/52, A61P 3/10

(54) **VECTEUR POUR ADMINISTRATION PAR VOIE ORALE**
SYSTEM ZUR ORALEN VERABREICHUNG
VECTOR FOR ORAL ADMINISTRATION

(30) Priorité: 23.04.2003 FR 0304976
(43) Date de publication de la demande: 15.03.2006
(73) Titulaire: CENTRE NATIONAL DE LA RECHERCHE SCIENTIFIQUE -CNRS-, 75016 Paris (FR); Centre Européen d'Etude du Diabète, 67200 Strasbourg (FR)
(72) Inventeur: FRERE, Yves, F-67810 HOLTZHEIM (FR); DANICHER, Louis, F-67200 STRASBOURG (FR); BELCOURT, Alain, F-67200 STRASBOURG (FR)
(74) Mandataire: Domenego, Bertrand
(86) Numéro de dépôt international: PCT/FR2004/000974
(87) Numéro de publication internationale: WO 2004/096172

(56) Documents cités:
- WO-A-01/72278
- WO-A-97/47323
- WO-A-02/064112

## Description

La présente invention concerne un véhicule gastro-résistant destiné à l'administration par voie orale d'au moins une substance active sur le plan pharmacologique, des procédés de préparation dudit véhicule, ses utilisations, ainsi que les compositions pharmaceutiques qui les renferment.

Parmi les différentes voies d'administration de principes actifs dans le domaine thérapeutique, la voie orale et les administrations par injections sont de loin les plus utilisées. Cependant, les administrations par injection, méthode invasive, ne sont pas toujours facilement acceptées par les patients. De plus, de nombreux traitements thérapeutiques nécessitent plusieurs injections quotidiennes, ce qui rend le traitement lourd et peu commode, notamment pour les patients qui doivent disposer en permanence du matériel complet pour injection et qui, bien souvent doivent pratiquer eux-mêmes leurs injections.

D'autres voies d'administration, comme les voies nasales, pulmonaires (de types spray, aérosols, gouttes, etc.) ou autres se sont souvent avérées peu efficaces dans le traitement thérapeutique d'affections, autres que les affections topiques nasales ou pulmonaires elles-mêmes.

Il serait donc très avantageux de pouvoir s'affranchir le plus possible des voies d'administration nasale, pulmonaire, par injection, et autres, et de les remplacer par des administrations par voie orale, plus physiologiques et plus confortables, pour le plus grand nombre possible de principes actifs. L'administration par injection présente toutefois des avantages bien connus dans ce domaine, et en particulier celui de permettre une action très rapide du principe actif, puisque celui-ci est directement ou quasiment instantanément disponible dans la circulation sanguine.

Un autre avantage considérable de l'administration par injection est que de nombreux principes actifs ne peuvent aujourd'hui être administrés en tant que tels par voie orale, lesdits principes actifs étant dégradés ou dénaturés, partiellement ou totalement, lors de l'ingestion (salive, suc gastrique, enzymes gastro-intestinales, etc.) avant de pouvoir parvenir dans la circulation sanguine. De tels principes actifs sont par exemple les composés de natures peptidiques ou protéiques, tels que les vaccins, les hormones (insuline par exemple), et autres.

Les avantages décrits ci-dessus permettent d'expliquer que les administrations par injections restent encore aujourd'hui très utilisées au détriment de l'administration par voie orale. En effet, malgré les très nombreux travaux effectués jusqu'à ce jour visant à administrer par voie orale des composés sensibles aux conditions du tractus gastro-intestinal (pH, contrainte mécanique, équipements enzymatiques divers), il n'a pas été possible de combiner de manière satisfaisante le confort de la prise de médicament par voie orale et les avantages d'une injection (absence de dénaturation ou de dégradation, ou faible dénaturation ou dégradation du principe actif, rapide disponibilité dans le sang).

De nombreux chercheurs ont cependant tenté de résoudre ces problèmes et les solutions mises en évidence suite à ces travaux sont diverses et variées. Un des axes de recherche consiste à protéger les substances actives par un revêtement gastro-résistant de manière à diminuer voire éviter la dénaturation, voire la dégradation de la substance active lors du passage dans l'estomac.

C'est ainsi que E. A. Hosny et coll. (Pharmaceutica Acta Helvetiæ, 72, (1997), 203-207) ont proposé d'améliorer la biodisponibilité de l'insuline administrée par voie orale en la plaçant en présence de cholate de sodium et en protégeant ce mélange par des capsules enrobées. La substance active est ainsi protégée dans l'estomac et se trouve libérée dans l'intestin. Les résultats sont cependant qualifiés seulement de "prometteurs" : une administration de capsules directement dans l'estomac de rats rendus hyperglycémiques a conduit à une activité hypoglycémiante plus faible que lors d'une administration, en quantité équivalente, par injection sous cutanée.

N. Shimono et coll., (International Journal of Pharmaceutics, 245, (2002), 45-54) ont envisagé d'utiliser des enveloppes polymères hydrophobes résistantes aux attaques gastriques, de manière que la substance active soit libérée spécifiquement dans le côlon dans le but d'en traiter les affections. Ces enveloppes sont par conséquent résistantes à l'estomac ainsi qu'à l'intestin grêle, ce qui semble être un handicap pour que la substance active parvienne à la circulation sanguine. En effet, il semble nécessaire que la substance active soit libérée dans l'intestin grêle, puis traverse la paroi intestinale afin de pénétrer dans la circulation sanguine, éventuellement via le liquide interstitiel.

Un autre axe de recherche a été de déterminer des formes galéniques permettant d'améliorer l'absorption intestinale de la substance active. Dans la publication de F. A. Dorkoosh et coll. (International Journal of Pharmaceutics, 247, (2002), 47-55), sont décrits des systèmes à base de polymères hydrogel superporeux contenant de l'insuline, mis sous forme de minitablettes. Bien qu'une administration orale soit envisagée dans ces travaux, aucun test n'a été réalisé en utilisant cette voie. De plus, les résultats présentés, au demeurant peu fiables, ne permettent pas de conclure quant à une efficacité pharmacologique probante de l'insuline administrée par ces systèmes.

D'autres publications, présentent diverses formes galéniques administrables par voie orale, par exemple gélules, capsules (microcapsules ou nanocapsules), systèmes matriciels, voire des systèmes moins conventionnels comme des systèmes « éponge » (R. Bodmeier et coli., Pharmaceutical Research, 6(5), (1989), 413-417).

Lors de l'administration par voie orale, un troisième axe de recherche vise l'augmentation de la quantité de substance active absorbée à travers la barrière intestinale par adhésion des composés administrés sur les microvillosités intestinales, à savoir la muco-adhésion. Par exemple, G. Ponchel et coll. (European Journal of Pharmaceutics and Biopharmaceutics, 44 (1997), 25-31), se sont intéressés à la muco-adhésion de véhicules contenant une ou plusieurs substances actives. Cependant, le passage de ces véhicules au travers de l'épithélium intestinal est présenté comme secondaire. L'amélioration de la biodisponibilité est uniquement présentée comme résultant de la muco-adhésion et de la longue période de temps d'adhésion aux muqueuses

Dans le brevet US 5,206,219, est décrit une composition pharmaceutique possédant un revêtement entérique adapté pour l'administration orale comprenant un co-solvant pharmaceutique polyol combiné avec un solvant pharmaceutique lipidique formant émulsion au contact des microvillosités intestinales. Cependant, les problèmes du passage de la paroi intestinale et de la libération de la substance active dans le sang, éventuellement via le liquide interstitiel, ne sont pas évoqués. De même, il n'est pas fait mention de la bioassimilation des composants de cette composition pharmaceutique

Une autre solution a été décrite dans la publication de G. P. Carrino et coll. (Journal of Controlled Release, 65, (2000), 261-269), dans laquelle de l'insuline couplée à du zinc est encapsulée dans des nanosphères possédant un revêtement poly(lactide-co-glycolide), lesquelles nanosphères passent à travers l'épithélium intestinal dans une période allant de 1 heure à 6 heures après administration par voie orale. Le poly(lactide-co-glycolide) étant un polymère relativement hydrophile, la muco-adhésion sur les villosités intestinales n'est pas satisfaisante. En conséquence, les auteurs ont ajouté de l'oxyde de fer afin d'augmenter cette muco-adhésion. Néanmoins, même en utilisant un tel système, les résultats obtenus sont loin d'être satisfaisants, l'efficacité pharmacologique représentant seulement 11,4 % par rapport à l'activité pharmacologique d'une administration d'insuline en quantité équivalente par voie intra péritonéale.

De manière paradoxale, malgré le nombre de travaux réalisés sur le sujet, aucune solution apportée n'est véritablement satisfaisante. Il subsiste aujourd'hui encore un besoin pour des systèmes d'administration par voie orale.

Ainsi, un premier objectif de l'invention consiste à fournir un système d'administration par voie orale de substances actives du point de vue pharmacologique possédant des caractéristiques de disponibilité desdites substances dans le sang comparables aux caractéristiques d'injection directe dans le sang desdites substances actives.

Un autre objectif consiste à fournir des systèmes d'administration permettant la libération dans le sang d'une ou plusieurs substances actives sur le plan pharmacologique qui soient peu ou pas dénaturées ou dégradées lors d'une administration par voie orale.

Un autre objectif consiste encore à fournir des systèmes d'administration permettant à une ou plusieurs substances actives sur le plan pharmacologique de traverser la paroi intestinale sans être dénaturées ou dégradées substantiellement.

Un des objectifs consiste également à fournir des systèmes d'administration permettant à une ou plusieurs substances actives sur le plan pharmacologique ayant traversé la paroi intestinale d'être disponibles dans le sang, éventuellement après passage dans le liquide interstitiel.

Comme autre objectif, la présente invention consiste à fournir des systèmes d'administration par voie orale d'une ou plusieurs substances actives sur le plan pharmacologique dans lesquels la ou lesdites substances actives ne sont pas ou peu dénaturées ou dégradées lors du passage dans le tractus gastro-intestinal, lors du passage au travers de la paroi intestinale, et qui permettent une disponibilité immédiate, retardée ou prolongée dans le sang.

D'autres objectifs encore deviendront apparents dans la description de la présente invention qui suit.

Il a à présent été découvert que les objectifs décrits ci-dessus peuvent être atteints en totalité ou en partie, grâce à des véhicules gastro-résistants, tels que ceux définis ci-après.

Ainsi, et selon un premier aspect, la présente invention concerne un véhicule gastro-résistant pour administration par voie orale d'au moins une substance, hydrophile, active sur le plan pharmacologique permettant le passage de ladite substance hydrophile active de la lumière de l'intestin dans le sang, éventuellement via le liquide interstitiel, sans dénaturation ou dégradation substantielle de ladite substance, ledit véhicule gastro-résistant comprenant une pluralité de vecteurs comprenant une matrice hydrophile comprenant comme constituant principal un poly(lactate-co-glycolate) ou un mélange comprenant un poly(lactate-co-glycolate) renfermant une ou plusieurs substances hydrophiles actives, la surface externe de ladite matrice étant modifiée par des espèces chimiques lui conférant un caractère lipophile, lesdits vecteurs étant dispersés dans un milieu lipophile lui-même contenu dans une protection gastrique de nature solide.

L'administration d'une ou plusieurs substances actives par voie orale consiste en fait à apporter, de la bouche vers le sang, la ou les substances actives, sans que celles-ci soient substantiellement dénaturées ou dégradées. On entend ainsi que la dose de substance active administrée par voie orale doit pouvoir se retrouver de manière substantiellement qualitative et quantitative dans le sang. Par "substantiellement quantitative et qualitative", on entend que la quantité de substance active dans le sang par rapport à la quantité de substance active administrée par voie orale doit être supérieure à 50%, de préférence supérieure à 65%, avantageusement supérieure à 80%, de manière optimale, supérieure à 90%.

Ainsi, lors d'une administration par voie orale, une substance active du point de vue pharmacologique doit franchir tous les obstacles présents dans un organisme avant de parvenir dans le flux sanguin, et ce, sans subir de dénaturations ou de dégradations substantielles. Les principaux obstacles et difficultés, pris en compte dans le cadre de la présente invention, sont tout d'abord le passage de la substance active dans l'estomac, le séjour dans la lumière intestinale, l'adhésion aux microvillosités présentes dans l'intestin, le passage de l'intestin vers le sang, éventuellement via le liquide interstitiel.

Les inventeurs ont considéré en premier lieu que la ou les substances actives administrées par voie orale doivent parvenir sans dénaturation ou dégradation substantielle dans le sang ou dans le liquide interstitiel. S'agissant du liquide interstitiel, celui-ci présente une valeur de pH comprise entre environ 6,5 et environ 7,5, plus particulièrement entre environ 7,2 et environ 7,3, et présente une force ionique non négligeable.

En considérant que les caractéristiques du sang et du milieu interstitiel sont différentes des autres organes (estomac, intestin entre autres) traversés depuis la cavité buccale, les inventeurs ont imaginé un vecteur pour la substance active, vecteur qui puisse être biocompatible et bioassimilable ou métabolisable.

Ainsi, ce vecteur doit être hydrophile, pour être compatible avec les divers fluides corporels (liquide lymphatique, liquide interstitiel, sang, ...). Le vecteur doit en outre libérer rapidement, ou de manière prolongée ou de manière retardée, la ou les substances actives à un pH compris entre environ 6,5 et 7,5, idéalement entre environ 7,2 et 7,3, pour permettre auxdites substances d'être ensuite disponibles dans le sang, à travers le vecteur et/ou après dégradation de celui-ci.

Sans entrer dans des considérations mécanistiques complexes, il est envisagé que le vecteur soit dégradé par les enzymes présentes dans le milieu (lysozyme, estérases, glycosidases, ...). La dégradation du vecteur permettra la libération immédiate, prolongée ou retardée de la ou des substances actives dans le liquide interstitiel pour atteindre la circulation sanguine. Une fois dans le sang, la ou les substances actives interagiront avec les sites d'intérêt, ou seront transportées jusqu'aux sites ou organes, afin de produire l'effet pharmacologique désiré.

De plus, ce vecteur doit être optimisé de sorte que sa nature hydrophile soit modifiée afin de la rendre compatible avec la paroi de l'intestin. En effet, la paroi de l'intestin est un milieu essentiellement lipophile dont le pH est supérieur à environ 7,8. Il convient par conséquent de modifier le vecteur décrit ci-dessus de sorte qu'il soit essentiellement lipophile au voisinage et sur la paroi intestinale, qu'il présente une muco-adhésion relativement bonne et enfin qu'il résiste à l'environnement entérique (milieu basique de pH supérieur à environ 7,8, présence d'enzymes de dégradation, ...).

Les inventeurs ont découvert qu'il était possible de combiner l'ensemble des critères mentionnés ci-dessus. Pour ce faire, un traitement de surface est appliqué à une matrice à caractère hydrophile, comprenant la ou les substances actives, de façon à lui conférer un caractère essentiellement lipophile. L'ensemble substance(s) active(s), matrice hydrophile et traitement de surface conférant le caractère lipophile définit le vecteur lipophile selon la présente invention.

Par "caractère hydrophile" ou "caractère essentiellement hydrophile", on entend une matrice à caractère uniquement hydrophile, ou encore à caractère lipophile et hydrophile, le caractère hydrophile étant dans ce cas prédominant par rapport au caractère lipophile dans le milieu d'intérêt. De même par "caractère lipophile" ou "caractère essentiellement lipophile", on entend un vecteur à caractère uniquement lipophile, ou encore à caractère lipophile et hydrophile, le caractère lipophile étant dans ce cas prédominant par rapport au caractère hydrophile dans le milieu d'intérêt. Dans la suite du présent exposé, on comprendra comme équivalents les termes "matrice à caractère (essentiellement) hydrophile" et "matrice hydrophile" d'une part, et "vecteur à caractère (essentiellement) lipophile" et "vecteur lipophile" d'autre part.

Le traitement de surface défini ci-dessus consiste généralement à modifier la surface de la matrice hydrophile par une ou plusieurs espèces chimiques biocompatibles susceptibles de se désolidariser de celle-ci lors de son passage de la lumière de l'intestin vers le sang, éventuellement via le liquide interstitiel.

Le vecteur selon l'invention est donc un composite susceptible de répondre à l'ensemble des critères énoncés plus haut et comprend une matrice hydrophile, renfermant une ou plusieurs substances actives, dont la surface a été traitée de manière à lui conférer un caractère lipophile.

Le constituant principal de la matrice hydrophile du vecteur est choisi parmi les poly(lactate-co-glycolate)s (dénommés PLGA dans la suite), ainsi que leurs copolymères et leurs mélanges avec les polylactates, les polymères ou copolymères à base d'acide hyaluronique, de chitosane, d'amidon, de dextran, et d'autres. Il peut être envisagé par exemple que le constituant principal de la matrice soit un mélange PLGA - acide hyaluronique, PLGA - chitosane, PLGA - amidon ou encore PLGA - dextran, ou d'autres mélanges.

D'autres constituants de la matrice hydrophile sont bien entendu envisageables, pour autant qu'ils confèrent à ladite matrice un caractère essentiellement hydrophile et biocompatible et/ou bioassimilable ou métabolisable. Ces constituants doivent en outre être compatibles avec la ou les substances actives renfermées dans le vecteur de sorte qu'elles ne soient pas substantiellement dénaturées ou dégradées avant d'être disponibles dans le sang.

La matrice définie ci-dessus est modifiée par des espèces chimiques susceptibles de moduler son caractère essentiellement hydrophile afin de lui conférer un comportement essentiellement lipophile, compatible avec ses propriétés requises d'adhésion aux microvillosités et de passage au travers de la paroi intestinale. Des espèces chimiques convenables sont connues de l'homme du métier, dont certaines sont par exemple décrites par H. Takeuchi et coll. (Adv. Drug Delivery Rev., 47, (2001), 39-54).

Ces espèces chimiques sont par exemple choisies parmi les paraffines, les lécithines, les acides aminés, les acides gras en général ainsi que leurs dérivés (esters et autres, par exemple les stéarates, les glycérides) les benzyles, les inositol-phosphates (IP), les glycérol-phosphates, les polymères lipophiles, et autres, ainsi que leurs mélanges.

La surface externe de la matrice hydrophile est soumise à un traitement par les espèces chimiques définies précédemment, qui sont ainsi rendues solidaires de la matrice hydrophile par des liaisons dites «faibles», de manière que celles-ci puissent être désolidarisées de la matrice par contact avec les microvillosités présentes dans l'intestin et lors du passage au travers de la barrière intestinale. La désolidarisation des espèces chimiques de la matrice lui permet ainsi de retrouver son caractère essentiellement hydrophile.

Le procédé de traitement de la surface de la matrice hydrophile peut être de tout type connu dans ce domaine, permettant l'adhésion, par un ou plusieurs types de liaisons définies ci-dessus, des espèces chimiques à la surface externe de la matrice hydrophile. Un procédé de traitement pourra par exemple être de type par trempage dans une solution contenant lesdites espèces chimiques, pulvérisation desdites substances, enrobage, pelliculage, par traitement par plasma froid, etc... Ce traitement de surface peut en outre être réalisé de manière à conduire à un revêtement de type monocouche ou multi-couches.

Le traitement de surface de la matrice peut être opéré avant ou après l'introduction de la ou des substances actives dans la matrice. Dans certains modes de réalisation de l'invention, par exemple lorsque la matrice se présente sous la forme d'une membrane de capsule, le traitement de surface peut être également effectué pendant la préparation proprement dite de la matrice.

Après passage au travers de la paroi intestinale, et désolidarisation des espèces chimiques, la matrice retrouve, par ce biais, le caractère hydrophile requis dans le sang et/ou le liquide interstitiel, et qui étaient les siennes avant traitement par les espèces chimiques définies précédemment. Les liaisons faibles envisagées plus haut peuvent être de tout type connu de l'homme du métier et par exemple des liaisons de nature électrostatique et/ou ionique et/ou de type liaison hydrogène ou autres.

Il convient en outre que le vecteur (ensemble substance(s) active(s), matrice et espèces chimiques) présent dans la lumière de l'intestin possède une taille et une forme permettant le passage physique dudit vecteur au travers de la membrane intestinale. En particulier, la taille dudit vecteur sera avantageusement comprise entre environ 10 nm et environ 10 µm, de préférence entre environ 100 nm et environ 500 nm, de préférence encore entre environ 200 nm et environ 300 nm. Une taille supérieure à 10 µm est moins préférée car le vecteur ne pourrait plus traverser la paroi de l'intestin. De même, une taille inférieure à environ 10 nm est également moins préférée, la quantité de substance active transportée par le vecteur pouvant être trop faible.

La forme du vecteur n'a pas d'importance spécifique en soi pour autant qu'elle permette un passage aisé au travers de la paroi intestinale. Ainsi, le vecteur pourra se présenter sous toute forme connue, par exemple sphère, aiguille, ovoïde, etc., dont la plus grande dimension, facteur limitant du passage au travers de la paroi intestinale, est avantageusement comprise entre environ 10 nm et environ 10 µm, de préférence entre environ 100 nm et environ 500 nm, de préférence encore entre environ 200 nm et environ 300 nm.

Selon un mode de réalisation préféré de la présente invention, le vecteur se présente sous forme de sphères de diamètre compris avantageusement entre environ 10 nm et environ 10 µm, de préférence entre environ 100 nm et environ 500 nm, par exemple entre environ 200 nm et environ 300 nm.

Lorsque le vecteur se présente sous forme de sphères, celui peut être préparé selon les techniques classiques d'encapsulation de substances actives, telles que par exemple par coacervation, simple ou complexe, polycondensation interfaciale, nébulisation/séchage (spray-drying), enrobage en lit fluidisé (spray-coating), etc...

Le vecteur selon la présente invention comprend une matrice renfermant une ou plusieurs substances actives du point de vue pharmacologique. À ce titre, la matrice peut être conçue sous la forme d'un gel renfermant une substance active, ou plusieurs substances actives sous forme de mélange. Selon un autre aspect, la matrice se présente sous forme d'une capsule renfermant une, ou plusieurs substances actives sous forme de mélange. D'autres formes encore sont envisageables, par exemples des formes de type "éponge", ou toutes autres formes solides plus ou moins compactes et pouvant libérer par diffusion et/ou après dégradation, la ou les substances actives qu'elles renferment.

Selon un mode de réalisation préféré de la présente invention, le vecteur est une capsule à caractère essentiellement lipophile, dont la membrane constitue la matrice hydrophile. La capsule renferme une ou plusieurs substances actives ou encore un mélange de substances actives, la membrane de la capsule ayant été modifiée par une ou plusieurs substances chimiques lui conférant ainsi un caractère essentiellement lipophile.

Il doit être précisé que, outre la ou les substances actives, le vecteur peut également contenir tout excipient, charge, colorant, et autres appropriés, connus de l'homme du métier, et non toxiques du point de vue pharmacologique.

Le vecteur tel que défini précédemment, comprenant une matrice hydrophile et renfermant une ou plusieurs substances actives, matrice modifiée par des espèces chimiques lui conférant un caractère lipophile, possède alors les caractéristiques qui le rendent compatible avec le milieu intestinal. En particulier, le caractère lipophile du vecteur assurera la propriété de muco-adhésion nécessaire à l'ancrage du vecteur sur les microvillosités.

Pour être efficace sur le plan pharmacologique, ce vecteur complexe destiné à être administré par voie orale doit en outre présenter une forte résistance au milieu stomacal par lequel il va transiter avant de parvenir à l'intestin. L'estomac est en effet un organe dans lequel le pH est très acide (aux alentours de 2, voire inférieur). De plus les enzymes présentes (en particulier la pepsine) dans l'estomac peuvent dénaturer, endommager voire détruire complètement ledit vecteur et par là même la ou les substances actives qu'il renferme.

Par protection gastrique du vecteur, on entend tout véhicule capable de protéger ledit vecteur des contraintes physiologiques inhérentes à l'estomac, ces contraintes étant principalement le pH acide et les enzymes stomacales (pepsine). Bien entendu, les constituants du véhicule, ainsi que leurs produits de dénaturation ou de dégradation doivent être non toxiques pour l'organisme.

De tels véhicules sont déjà très largement connus dans le domaine (médicaments encapsulés par exemple, comme décrit dans "Encyclopedia of Pharmaceutical Technology", Marcel Dekker, (1992), J. Swarbrick and J. C. Boylan Editors, Enteric Coatings, pp. 189-200). Tout véhicule gastro-résistant de nature solide connu de l'homme du métier peut par conséquent être utilisé. De préférence, il peut être sous forme de gel, ou se présenter sous la forme d'un revêtement ou d'une capsule, et renfermer un ou plusieurs vecteurs tels que définis précédemment, eux-mêmes sous diverses formes, capsules, gels ou autres.

Selon un aspect préféré de la présente invention, le véhicule gastro-résistant se présente sous la forme d'une capsule contenant un ou plusieurs vecteurs tels que définis précédemment. Lesdits véhicules sous forme de capsule peuvent avantageusement être obtenus par des procédés de type coacervation, polycondensation interfaciale en milieu dispersé, ou autres. Bien entendu, tout autre procédé connu d'encapsulation peut être utilisé et/ou adapté en vue de préparer les véhicules de l'invention.

Parmi les constituants capables de résister aux contraintes physiologiques inhérentes à l'estomac, on peut citer en particulier les alginates, comme l'alginate de calcium, la carboxyméthylcellulose, et autres, ainsi que leurs mélanges. Le véhicule gastro-protecteur devra résister à un pH acide, notamment inférieur à 2 et plus particulièrement inférieur à 1,2 ainsi qu'aux attaques des enzymes gastriques.

Bien entendu, les constituants du véhicule doivent posséder comme caractéristiques de pouvoir être modifiés ou dégradés de manière spécifique dans la lumière de l'intestin, c'est-à-dire à un pH supérieur à environ 7,8 et en présence des enzymes entériques, afin de libérer le vecteur dans la lumière de l'intestin.

En outre, le véhicule gastro-résistant renferme un milieu lipophile, dans lequel le ou les vecteurs définis précédemment sont présents. Ce milieu lipophile peut être sous forme solide, liquide ou encore sous forme de gel. Le milieu lipophile peut être constitué de tout composé lipophile connu en soi et non toxique du point de vue pharmacologique. Le composé lipophile envisagé peut par exemple être choisi parmi les huiles organiques ou minérales, végétales ou animales, par exemple, l'huile d'olive, l'huile de foie de morue, huiles silicone, et autres, ainsi que leurs mélanges.

Le vecteur, comprenant la matrice hydrophile modifiée par des espèces chimiques lui conférant le caractère lipophile, permet d'empêcher toute fuite de substance active dans le flux intestinal, également hydrophile. Les modifications lipophiles, en plus de leurs fonctions muco-adhésives, jouent ainsi un rôle clé en temps que barrière hydrophobe à la matière active.

Ainsi, le vecteur selon la présente invention, doté d'une protection gastrique, peut être utile pour l'administration par voie orale de toute substance active sur le plan pharmacologique susceptible d'être modifiée, affectée, remaniée, métabolisée, stockée, dénaturée ou dégradée dans le tractus gastro-intestinal, lors d'une administration conventionnelle directe par voie orale. Les véhicules gastro-résistants comprenant plusieurs vecteurs tels qu'ils viennent d'être définis dans la description ci-dessus font partie de la présente invention.

Un autre objet de la présente invention concerne par conséquent l'utilisation du véhicule gastro-résistant selon la présente invention pour permettre une administration par voie orale et de manière efficace sur le plan pharmacologique de substances actives de nature peptidique ou protéique, c'est-à-dire se caractérisant par une ou plusieurs séquences d'acides aminés. Les substances actives comprises dans le vecteur de la présente invention peuvent être ainsi de nature diverse et variée.

On pourra citer à titre d'exemples non limitatifs les hormones de nature peptidique et notamment l'insuline. L'utilisation du vecteur selon l'invention ne se limite cependant pas à ces substances actives de nature peptidique ou protéique et toute autre substance active du point de vue pharmacologique risquant une dégradation ou une dénaturation est également comprise dans le champ de la présente invention.

Comme énoncé dans la présente description, le véhicule gastro-résistant selon l'invention sera utilisé en vue de permettre l'administration par voie orale d'une ou plusieurs substances actives et notamment le transfert de la ou desdites substances actives de la lumière de l'intestin vers la circulation sanguine, éventuellement via le liquide interstitiel.

La présente invention concerne également les compositions pharmaceutiques comprenant un ou plusieurs vecteurs, identiques ou différents, dotés d'au moins une protection gastrique, avec tout excipient, charges, colorants, liants, autres substances actives, édulcorants, arômes, etc., non toxiques et bien connus de l'homme du métier.

Ainsi, et selon un autre objet de la présente invention, le véhicule gastro-résistant selon la présente invention pourra être utile pour la préparation d'un médicament administrable par voie orale en thérapeutique humaine ou vétérinaire, possédant des propriétés curatives et/ou préventives et/ou permettant le diagnostic, et en particulier pour l'administration par voie orale des substances actives de nature peptidique ou protéique, y compris les vaccins.

Le véhicule gastro-résistant selon l'invention trouve par exemple un emploi tout à fait intéressant pour la préparation de produits pharmaceutiques pour administration par voie orale chez l'homme ou l'animal. Une utilisation particulièrement préférée concerne la préparation de produits pharmaceutiques destinés à divers traitements, par exemple, et de manière non limitative, ceux choisis parmi le traitement du diabète par voie orale, et en particulier du diabète de type 1 insulinodépendant (vectorisation de l'insuline), la vaccination par voie orale (vectorisation de vaccins), et les traitements hormonaux (vectorisation d'hormones de toutes natures), pour ne citer que quelques exemples non limitatifs.

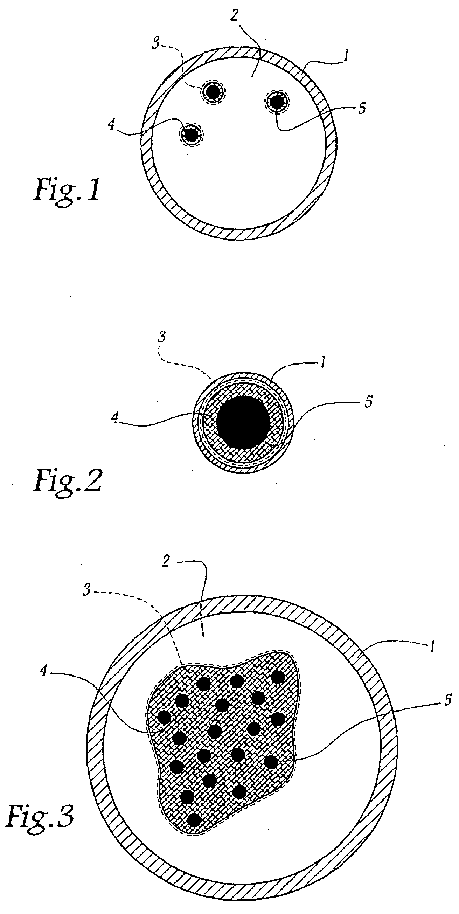

Les figures 1 à 3 annexées présentent quelques exemples non limitatifs de réalisation des vecteurs selon la présente invention.
- La figure 1 représente plusieurs vecteurs (3,4,5) dispersés dans un milieu lipophile (2) et encapsulés dans une protection gastrique (1). Chaque vecteur (3,4,5) est constitué d'une substance active (5) encapsulée dans une matrice hydrophile (4) modifiée par des espèces chimiques (3) conférant le caractère lipophile audit vecteur (3,4,5).
- La figure 2 représente un vecteur (3,4,5) constitué d'une substance active (5) encapsulée dans une matrice hydrophile (4) modifiée par des espèces chimiques (3) conférant le caractère lipophile audit vecteur (3,4,5). Le vecteur (3,4,5) est directement encapsulé dans une protection gastrique (1).
- La figure 3 représente un vecteur (3,4,5) constitué d'une matrice hydrophile (4) sous forme de gel dans lequel a été dispersée une substance active (5). Ce gel est modifié par des espèces chimiques (3) conférant le caractère lipophile audit vecteur (3,4,5). Le vecteur (3,4,5) est placé dans un milieu lipophile (2) lui-même encapsulé dans une protection gastro-résistante (1).

Les exemples suivants présentent des modes de réalisation possibles pour la présente invention, sans toutefois la limiter en aucune façon. Il doit en outre être compris que des modifications peuvent être apportées à ces exemples de réalisation, les vecteurs et véhicules obtenus restant compris dans le champ de la présente invention.

### EXEMPLE 1

### Exemple de synthèse d'une matrice sous forme de capsule hydrophile, renfermant de l'insuline

Des capsules sont synthétisées suivant le procédé d'émulsion multiple avec évaporation ou extraction de solvant.

L'émulsion multiple, eau dans huile dans eau, est réalisée en deux temps :
- dans un premier temps une émulsion simple, eau dans huile, est obtenue par dispersion rapide (agitation: homogénéiseur Ultra-Turrax® ; 15 000 tr/mn ; 3 fois 10s ; 0°C) de 50 µL d'une solution aqueuse de principe actif (insuline ; NovoRapid ; 100U/mL) dans 5 mL d'une solution organique (dichlorométhane) contenant 100 mg de polymère biocompatible (poly(L-Lactate); Fluka ; Mw = 152 000 ou poly(D,L-lactate-co-glycolate) ; Aldrich ; Mw = 50 000 - 75 000 ; fraction lactate/glycolate = 85/15 ou poly(D,L-lactate-co-glycolate) ; Aldrich ; Mw = 50 000 - 75 000 ; fraction lactate/glycolate = 50/50),
- dans un second temps, cette première émulsion est dispersée (agitation : homogénéiseur Ultra-Turrax® ; 10 000 tr/mn ; 3 fois 15 s ; 0°C) dans 50 mL d'une solution aqueuse (1 % p/v) d'alcool polyvinylique (Mowiol 4-88 ; Hoechst ; Mw = 26 000).

Dans le cas du procédé par évaporation, l'émulsion multiple est diluée dans 150 mL d'une solution aqueuse (0,3% p/v) d'alcool polyvinylique (Mowiol 4-88 ; Hoechst ; Mw = 26 000), cette solution est agitée sous pression réduite (évaporateur rotatif ; 500 mm/Hg ; 3h ; 25°C) pour permettre l'évaporation du solvant organique.

Dans le cas du procédé par extraction, l'émulsion multiple est diluée dans 200 mL d'une solution aqueuse (2% v/v) d'isopropanol, cette solution est agitée (agitation: magnétique; 250 tr/mn; 1h ; 25°C) pour permettre l'extraction du solvant organique.

Après évaporation ou extraction du solvant organique, les capsules sont récupérées par filtration, lavées à l'eau, centrifugées, lyophilisées puis conservées au froid (4°C).

### EXEMPLE 2

### Exemple de synthèse d'un vecteur constitué d'une matrice sous forme d'éponge comprenant de l'insuline

Des matrices de type "éponge" sont synthétisées suivant le procédé de coacervation complexe.

Dans 100 mL d'une phase organique (huile minérale ; Aldrich) contenant un tensio-actif (Span®80 ; Aldrich ; 1% p/v) sont dispersées (agitation : homogénéiseur Ultra-Turrax® ; 15 000 tr/mn ; 0°C), simultanément mais séparément, deux solutions aqueuses, l'une (5 mL ; 1% p/v dans l'eau) de hyaluronate de sodium (à partir de Streptococcus Equi ; Fluka), l'autre (5 mL ; 1% p/v dans l'acide acétique 0,1 N) de chitosane (à partir de carapaces de crabes ; Fluka ; Mw = 150 000).

L'ajout est réalisé au moyen d'une seringue (diamètre intérieur de l'aiguille : 0,6 mm) et d'un pousse seringue (0,2 mL/mn).

La dispersion est ensuite agitée (agitation magnétique ; 200 tr/mn ; 12 h ; 40°C) pour permettre la formation des éponges.

Les particules sont séparées de la phase organique par centrifugation, lavées au cyclohexane (3 fois 100 mL), filtrées puis lyophilisées.

Les éponges sont immergées (12 h ; 25°C) dans une solution aqueuse de principe actif (insuline ; NovoRapid ; 100 U/mL), filtrées, rincées rapidement à l'eau stérilisée, lyophilisées puis conservées au froid (4°C).

Les éponges (à caractère essentiellement hydrophile) sont trempées dans une solution d'acides gras, de manière à obtenir des vecteurs à caractère essentiellement lipophile.

### EXEMPLE 3

### Exemple de synthèse de véhicules contenant les vecteurs de l'exemple 2

Les véhicules sont synthétisés suivant le procédé de coacervation.

Les vecteurs (100 mg) obtenus à l'exemple 2 et contenant le principe actif (insuline ; NovoRapid ; 100 U/mL) sont hydratés, filtrés puis dispersées (agitation : magnétique ; 200tr/mn ; 5 mn) dans une phase organique (huile d'olive ; 1 mL) avec du carbonate de calcium réduit en poudre fine (100 mg).

Cette phase organique est ajoutée au moyen d'une seringue (diamètre intérieur de l'aiguille : 1,2 mm) et d'un pousse seringue (0,2 mL/mn) dans 50 mL d'une phase aqueuse (0,25% p/v) d'alginate de sodium (Lancaster) et (1% v/v) d'acide acétique agitée en continu (agitation : mécanique (hélice) ; 300 tr/mn ; 1 h ; 25°C).

La solution d'alginate est diluée par un ajout de 200 mL d'eau permutée. Les véhicules sont récupérés par filtration, transférés dans une solution aqueuse (1,3% p/v) de chlorure de calcium. Après 15 mn d'incubation à température ambiante, les véhicules sont filtrés, rincés à l'eau permutée puis conservés dans de l'eau au froid (4°C).

## Revendications

1. Véhicule gastro-résistant pour administration par voie orale d'au moins une substance active hydrophile sur le plan pharmacologique permettant le passage de ladite substance active hydrophile de la lumière de l'intestin dans le sang, éventuellement via le liquide interstitiel, sans dénaturation ou dégradation substantielle de ladite substance, ledit véhicule gastro-résistant comprenant une pluralité de vecteurs comprenant une matrice hydrophile comprenant comme constituant principal un poly(lactate-co-glycolate) ou un mélange comprenant un poly(lactate-co-glycolate) renfermant une ou plusieurs substances actives hydrophiles, la surface externe de ladite matrice étant modifiée par des espèces chimiques lui conférant un caractère lipophile, lesdits vecteurs étant dispersés dans un milieu lipophile lui-même contenu dans une protection gastrique de nature solide.

2. Véhicule selon la revendication 1, **caractérisé en ce que** le vecteur est biocompatible et bioassimilable ou métabolisable à un pH compris entre environ 6,5 et 7,5, idéalement entre environ 7,2 et 7,3.

3. Véhicule selon l'une quelconque des revendications précédentes, **caractérisé en ce que** le constituant principal de la matrice hydrophile est choisi parmi les mélanges PLGA - acide hyaluronique, PLGA - chitosane, PLGA - amidon ou PLGA - dextran.

4. Véhicule selon l'une quelconque des revendications précédentes, **caractérisé en ce que** les espèces chimiques sont choisies parmi les paraffines, les lécithines, les acides aminés, les acides gras en général ainsi que leurs dérivés (esters et autres, par exemple les stéarates, les glycérides) les benzyles, les inositol-phosphates (IP), les glycérol-phosphates, les polymères lipophiles, et autres, ainsi que leurs mélanges.

5. Véhicule selon l'une quelconque des revendications précédentes, **caractérisé en ce que** les espèces chimiques sont fixées sur la matrice hydrophile par des liaisons faibles.

6. Véhicule selon la revendication 5, **caractérisé en ce que** les liaisons faibles sont des liaisons de nature électrostatique et/ou ionique et/ou de type liaison hydrogène.

7. Véhicule selon l'une quelconque des revendications précédentes, **caractérisé en ce que** la plus grande dimension des vecteurs est comprise entre environ 10 nm et environ 10 µm, de préférence entre environ 100 nm et environ 500 nm, de préférence encore entre environ 200 nm et environ 300 nm.

8. Véhicule selon la revendication 7, **caractérisé en ce que** les vecteurs se présentent sous forme de sphères de diamètre compris entre environ 10 nm et environ 10 µm, de préférence entre environ 100 nm et environ 500 nm, par exemple entre environ 200 nm et environ 300 nm.

9. Véhicule selon l'une quelconque des revendications précédentes, **caractérisé en ce qu'**il comprend une matrice sous forme d'un gel renfermant la ou lesdites substances actives ou encore un mélange de substances actives.

10. Véhicule selon l'une quelconque des revendications 1 à 9, **caractérisé en ce qu'**il comprend une matrice sous forme d'une capsule renfermant la ou lesdites substances actives ou encore un mélange de substances actives.

11. Véhicule selon la revendication 10, pour lequel la protection gastrique est sous forme de gel, ou se présente sous la forme d'un revêtement ou d'une capsule.

12. Véhicule selon l'une quelconque des revendications 1 à 9, pour lequel la protection gastrique se présente sous la forme d'une capsule.

13. Véhicule selon l'une quelconque des revendications 1 à 12, **caractérisé en ce que** la protection gastrique comprend des constituants choisis parmi les alginates, comme l'alginate de calcium, la carboxyméthylcellulose, et autres, ainsi que leurs mélanges.

14. Véhicule selon l'une quelconque des revendications 1 à 13, **caractérisé en ce que** le composé lipophile est choisi parmi les huiles organiques ou minérales, végétales ou animales, et leurs mélanges.

15. Véhicule selon l'une quelconque des revendications précédentes, **caractérisé en ce que** la substance active hydrophile est choisie parmi les substances susceptibles d'être dénaturées ou dégradées lors d'une administration directe par voie orale.

16. Véhicule selon l'une quelconque des revendications précédentes, **caractérisé en ce que** la substance active hydrophile est de nature peptidique ou protéique.

17. Véhicule selon l'une quelconque des revendications précédentes, **caractérisé en ce que** la substance active hydrophile est l'insuline.

18. Composition pharmaceutique comprenant au moins un Véhicule gastro-résistant tel que défini dans l'une quelconque des revendications 1 à 17.

19. Utilisation d'un Véhicule gastro-résistant selon l'une quelconque des revendications 1 à 17, en vue d'obtenir un médicament actif par voie orale en thérapeutique humaine ou vétérinaire et possédant des propriétés curatives et/ou préventives et/ou permettant le diagnostic.

20. Composition pharmaceutique selon la revendication 18 pour son utilisation dans le traitement du diabète de type 1.

21. Composition pharmaceutique selon la revendication 18 pour son utilisation dans le la vaccination par voie orale.

## Patentansprüche

1. Ein magensaftresistentes Transportmittel für die orale Verabreichung mindestens einer aktiven, hydrophilen, pharmakologischen Substanz, das das Passieren der aktiven, hydrophilen Substanz vom Darmlumen in das Blut, unter Umständen durch die Gewebsflüssigkeit, ohne Denaturierung oder substantielle Zersetzung der Substanz, ermöglicht, wobei das magensaftresistente Transportmittel eine Vielzahl von Vektoren umfasst, die eine hydrophile Matrix umfassen, die als Hauptbestandteil ein Poly(Lactat-Co-Glycolat) oder eine Mischung umfassend ein Poly(Lactat-Co-Glycolat) enthaltend eine oder mehrere aktive, hydrophile Substanzen umfasst, wobei die äußere Oberfläche der Matrix modifiziert ist mit chemischen Spezies, die lipophil sind, wobei die Vektoren in einem lipophilen Milieu dispergiert sind, das selbst in einem festen Magensaftschutz enthalten ist.

2. Transportmittel gemäß Anspruch 1, **gekennzeichnet dadurch, dass** der Vektor biokompatibel und bioassimilierbar oder metabolisierbar bei einem pH zwischen ungefähr 6,5 und 7,5, idealerweise zwischen ungefähr 7,2 und 7,3 ist.

3. Transportmittel gemäß einem der voranstehenden Ansprüche, **gekennzeichnet dadurch, dass** der Hauptbestandteil der hydrophilen Matrix ausgewählt ist aus den Mischungen PLGA-Hyaluronsäure, PLGA-Chitosan, PLGA-Amidon oder PLGA-Dextran.

4. Transportmittel gemäß einem der voranstehenden Ansprüche, **gekennzeichnet dadurch, dass** die chemischen Spezies ausgewählt sind aus Paraffinen, Lecithinen, Aminosäuren, Fettsäuren im Allgemeinen sowie ihren Derivaten (Ester und andere, zum Beispiel Stearate, Glyceride), Benzylen, Inositolphosphaten (IP), Glycerin-Phosphaten, lipophilen Polymeren und anderen sowie ihren Mischungen.

5. Transportmittel gemäß einem der voranstehenden Ansprüche, **gekennzeichnet dadurch, dass** die chemischen Spezies mittels schwachen Bindungen auf der hydrophilen Matrix befestigt sind.

6. Transportmittel gemäß Anspruch 5, **gekennzeichnet dadurch, dass** die schwachen Bindungen elektrostatische und/oder ionische Bindungen und/oder Wasserstoffbrückenbindungen sind.

7. Transportmittel gemäß einem der voranstehenden Ansprüche, **gekennzeichnet dadurch, dass** das größte Ausmaß der Vektoren zwischen ungefähr 10 nm und ungefähr 10 µm, vorzugsweise zwischen ungefähr 100 nm und ungefähr 500 nm, noch bevorzugter zwischen ungefähr 200 nm und ungefähr 300 nm ist.

8. Transportmittel gemäß Anspruch 7, **gekennzeichnet dadurch, dass** die Vektoren sphärenförmig mit einem Durchmesser zwischen ungefähr 10 nm und ungefähr 10 µm, vorzugsweise zwischen ungefähr 100 nm und ungefähr 500 nm, beispielsweise zwischen ungefähr 200 nm und ungefähr 300 nm sind.

9. Transportmittel gemäß einem der voranstehenden Ansprüche, **gekennzeichnet dadurch, dass** es eine gelförmige Matrix enthaltend die aktive(n) Substanz(en) oder aber eine Mischung von aktiven Substanzen umfasst.

10. Transportmittel gemäß einem der Ansprüche 1 bis 9, **gekennzeichnet dadurch, dass** es eine kapselförmige Matrix enthaltend die aktive(n) Substanz(en) oder aber eine Mischung von aktiven Substanzen umfasst.

11. Transportmittel gemäß Anspruch 10, für das der Magensaftschutz gelförmig oder in Form einer Beschichtung oder Kapsel ist.

12. Transportmittel gemäß einem der Ansprüche 1 bis 9, für das der Magensaftschutz in Form einer Kapsel ist.

13. Transportmittel gemäß einem der Ansprüche 1 bis 12, **gekennzeichnet dadurch, dass** der Magensaftschutz Bestandteile ausgewählt aus Alginaten, wie Calciumalginat, Carboxymethylcellulose und anderen sowie ihren Mischungen umfasst.

14. Transportmittel gemäß einem der Ansprüche 1 bis 13, **gekennzeichnet dadurch, dass** die lipophile Verbindung ausgewählt ist aus organischen oder mineralischen Ölen, pflanzlich oder tierisch, und ihren Mischungen.

15. Transportmittel gemäß einem der voranstehenden Ansprüche, **gekennzeichnet dadurch, dass** die aktive, hydrophile Substanz ausgewählt ist aus Substanzen, die empfindlich sind gegen Denaturierung oder Zersetzung während einer direkten, oralen Verabreichung.

16. Transportmittel gemäß einem der voranstehenden Ansprüche, **gekennzeichnet dadurch, dass** die aktive, hydrophile Substanz peptid- oder proteinartig ist.

17. Transportmittel gemäß einem der voranstehenden Ansprüche, **gekennzeichnet dadurch, dass** die aktive, hydrophile Substanz Insulin ist.

18. Pharmazeutische Zusammensetzung umfassend mindestens ein magensaftresistentes Transportmittel, das gemäß einem der Ansprüche 1 bis 17 definiert ist.

19. Verwendung eines magensaftresistenten Transportmittels gemäß einem der Ansprüche 1 bis 17, um ein oral aktives Medikament für die Human- oder Veterinärtherapie und mit heilenden und/oder präventiven und/oder Diagnostikermöglichenden Eigenschaften zu erhalten.

20. Pharmazeutische Zusammensetzung gemäß Anspruch 18 für deren Verwendung bei der Behandlung von Typ I Diabetes.

21. Pharmazeutische Zusammensetzung gemäß Anspruch 18 für deren Verwendung bei der oralen Impfung.

## Claims

1. A gastro-resistant vehicle for the oral administration of at least one hydrophilic pharmacologically active substance that allows said active substance to pass from the intestinal lumen to the blood, optionally via the interstitial fluid, without any substantial denaturation or degradation of said substance, said gastro-resistant vehicle comprising a plurality of vectors comprising a hydrophilic matrix comprising as main constituent a poly(lactate-co-glycolate) or a mixture comprising a poly(lactate-co-glycolate) entrapping one or more hydrophilic active substances, the outer surface of said matrix being modified with one or more chemical species that give it an essentially lipophilic nature, said vectors being dispersed in a lipophilic medium itself contained in a gastric protection of solid nature.

2. The vehicle as claimed in claim 1, **characterized in that** it is biocompatible and bioassimilable or metabolizable at a pH of between approximately 6.5 and 7.5, ideally between approximately 7.2 and 7.3.

3. The vehicle as claimed in any one of the preceding claims, **characterized in that** the main constituent of the hydrophilic matrix is selected from mixtures of PLGA - hyaluronic acid, PLGA - chitosane, PLGA - starch, or PLGA - dextran.

4. The vehicle as claimed in any one of the preceding claims, **characterized in that** the chemical species are selected from paraffins, lecithins, amino acids, fatty acids in general and also derivatives thereof (esters and the like, for example stearates, glycerides), benzyls, inositol phosphates (IPs), glycerol phosphates, lipophilic polymers, and the like, and also mixtures thereof.

5. The vehicle as claimed in any one of the preceding claims, **characterized in that** the chemical species are attached to the hydrophilic matrix via weak bonds.

6. The vehicle as claimed in claim 5, **characterized in that** the weak bonds are bonds of electrostatic and/or ionic nature and/or of hydrogen bond type.

7. The vehicle as claimed in any one of the preceding claims, **characterized in that** its largest dimension is between approximately 10 nm and approximately 10 µm, preferably between approximately 100 nm and approximately 500 nm, more preferably between approximately 200 nm and approximately 300 nm.

8. The vehicle as claimed in claim 7, **characterized in that** it is in the form of spheres having a diameter of between approximately 10 nm and approximately 10 µm, preferably between approximately 100 nm and approximately 500 nm, for example between approximately 200 nm and approximately 300 nm.

9. The vehicle as claimed in any one of the preceding claims, **characterized in that** it comprises a matrix in the form of a gel containing said active substance(s) or else a mixture of active substances.

10. The vehicle as claimed in any one of claim 1 to 9, **characterized in that** it comprises a matrix in the form of a capsule containing said active substance(s) or else a mixture of active substances.

11. The vehicle as claimed in claim 10, for which the gastric protection is in the form of a gel, or is in the form of a coating or of a capsule.

12. The vehicle as claimed in any one of claim 1 to 9, for which the gastric protection is in the form of a capsule.

13. The vehicle as claimed in any one of claim 1 to 12, **characterized in that** the gastric protection comprises constituents selected from alginates, such as calcium alginate, carboxymethylcellulose and the like, and also mixtures thereof.

14. The vehicle as claimed in any one of claim 1 to 13, **characterized in that** the lipophilic compound is selected from organic or mineral, plant or animal oils, and mixtures thereof.

15. The vehicle as claimed in any one of the preceding claims, **characterized in that** the active substance is selected from substances capable of being denatured or degraded upon direct oral administration.

16. The vehicle as claimed in any one of the preceding claims, **characterized in that** the active substance is peptide or protein in nature.

17. The vehicle as claimed in any one of the preceding claims, **characterized in that** the active substance is insulin.

18. A pharmaceutical composition comprising at least one vehicle as defined in any one of the claims 1 to 17.

19. Use of gastro-resistant vehicle according to any one of the claims 1 to 17 for obtaining a medicament that is active when administered orally in human or veterinary therapy and that has curative and/or preventive properties and/or properties that allow diagnosis.

20. Composition according to claim 18 for its use in the treatment of Type 1 diabetes.

21. Composition according to claim 18 for its use in the oral immunization.
